# EUROPEAN PATENT SPECIFICATION

(11) **EP 3 431 598 B1**
(45) Date of publication and mention of the grant of the patent: **11.12.2024**
(21) Application number: 17766759.9
(22) Date of filing: 16.03.2017
(51) Int. Cl.: C12N 15/10

(54) **METHOD FOR COLLECTING NUCLEIC ACID**
VERFAHREN ZUR ENTNAHME VON NUKLEINSÄUREN
PROCÉDÉ DE COLLECTE D'ACIDE NUCLÉIQUE

(30) Priority: 17.03.2016 JP 2016053830
(43) Date of publication of application: 23.01.2019
(73) Proprietor: Toray Industries, Inc., Tokyo 103-8666 (JP)
(72) Inventor: SEKIGUCHI, Shota, Kamakura-shi Kanagawa 248-8555 (JP); SAWADA, Shinjiro, Kamakura-shi Kanagawa 248-8555 (JP); NAKAGAWA, Mai, Kamakura-shi Kanagawa 248-8555 (JP)
(74) Representative: Kador & Partner Part mbB
(86) International application number: PCT/JP2017/010553
(87) International publication number: WO 2017/159763

(56) References cited:
- JP-A- 2007 006 728
- JP-A- 2010 155 931
- JP-A- H11 313 670
- TW-A- 201 144 037
- US-A- 5 234 809
- LING QI ET AL: "Redispersible Hybrid Nanopowders: Cerium Oxide Nanoparticle Complexes with Phosphonated-PEG Oligomers", ACS NANO, vol. 2, no. 5, 1 May 2008 (2008-05-01), pages 879 - 888, XP055011458, ISSN: 1936-0851, DOI: 10.1021/nn700374d
- MOON IL KIM ET AL: "Ultrafast colorimetric detection of nucleic acids based on the inhibition of the oxidase activity of cerium oxide nanoparticles", CHEMICAL COMMUNICATIONS, vol. 50, no. 67, 1 January 2014 (2014-01-01), UK, pages 9577 - 9580, XP055628870, ISSN: 1359-7345, DOI: 10.1039/C4CC03841J
- SONG, W. ET AL.: "Ceria nanoparticles stabilized by organic surface coating activate the lysosome-autophagy system and enhance autophagic clearance", ACS NANO, vol. 8, no. 10, 2014, pages 10328 - 10342, XP055420915
- PAUTLER, R. ET AL.: "Attaching DNA to nanoceria: Regulating oxidase activity and fluorescence quenching.", ACS APPL. MATER. INTERFACES, vol. 5, no. 15, 2013, pages 6820 - 6825, XP055420918

## Description

### [TECHNICAL FIELD]

The present invention relates to a method of collecting a nucleic acid, a support comprising a water-soluble neutral polymer adsorbed on a cerium oxide surface thereof, and a kit for collecting a nucleic acid.

### [BACKGROUND ART]

The development of experimental techniques using nucleic acids has allowed for a search and analysis for a new gene. Screening tests and clinical tests using gene analysis have been performed in the medical field as well, for example, a human genome has been analyzed in order to identify a disease such as a cancer, and a pathogen genome has been analyzed in order to identify a pathogen infection.

For the target in gene analysis, not only long-chain nucleic acids such as a genome but also short-chain nucleic acids have attracted attention. miRNAs which were discovered in recent years are single-stranded RNAs of not less than 18 bases in length and not more than 25 bases in length, and are biosynthesized from pre-miRNAs of not less than 60 bases in length and not more than 90 bases in length. miRNAs are believed to be related to diseases because they have a function to control protein synthesis and gene expression, and thus have attracted attention as a target of gene analysis. A method such as a metagenomic diagnosis method, in which nucleic acid fragments of several hundred bases in length derived from pathogens in a clinical sample are analyzed comprehensively by a next-generation sequencer has also attracted attention as a new gene analysis method. It is recognized that the target of the current gene analysis has diversified more as the gene search has developed. Therefore, for the method of collecting a nucleic acid as well, in response to the target diversification in gene analysis, a method of collecting nucleic acids ranging from those of several dozen bases in length such as miRNAs to long-chain nucleic acids such as genomes has been demanded.

What is required first in performing gene analysis is a step of collecting nucleic acids from a biological sample. If the nucleic acids can be collected with a high purity and high yield, a highly sensitive gene detection and gene analysis can be attained in the detection reaction afterwards. Exemplary examples of methods of collecting nucleic acids include phenol-chloroform extraction, ethanol precipitation and nucleic acid adsorption on silica.

Among these, the most common method is the Boom method, as described in Patent Document 1, in which nucleic acids are adsorbed on a metal oxide containing silica, then eluted, and collected. This method is characterized by the concentration of the nucleic acids along with the collection of the nucleic acids from the nucleic acid-adsorbed silica by a centrifugation operation. However, Patent Document 2 describes that, when Boom method is used, a nucleic acid of not less than 300 bases in length and not more than 1000 bases in length exhibits less adsorption on silica compared to that of a longer nucleic acid. Thus, it is expected that the collection of pre-miRNAs or miRNAs of not more than 100 bases in length would be difficult. Since gene analysis is also utilized in the medical field, a method of collecting nucleic acids without any complicated operation or the use of an organic solvent is preferred.

Patent Document 3 describes, as a method without an organic solvent, a method of collecting a nucleic acid by adsorbing the nucleic acid using a magnetic inorganic component such as iron oxide as a support. However, in this method, because the method has a drawback in that an inorganic component is eluted into a collecting solution for a nucleic acid, thereby reducing the purity of collected nucleic acid, a method of coating a polymer or a metal oxide on an inorganic support is described as well.

Furthermore, there is a method, as another method without an organic solvent, in which a silica gel ion exchange column is used. Patent Document 4 describes a method of improving a nucleic acid collection quantity by allowing a silica gel support to be used for an ion exchange column to contain a particle with a high relative gravity, such as titania, ceria, zirconia and hafnia, to increase the column density. However, in the method of Patent Document 4, though the efficiency of collecting a plasmid can be improved, the fact that nucleic acids in the form of small fragments are not adsorbed on a support is described in the paragraph [0134], and it is therefore expected that it is difficult to collect pre-miRNA and miRNA which are very short-chain nucleic acids. TW201144037 teaches a porous filter column for purifying nucleic acids from a biological sample.

### [PRIOR ART REFERENCES]

### [PATENT DOCUMENTS]

[Patent Document 1] US5,234,809 B
[Patent Document 2] JP 2011-522529 A
[Patent Document 3] JP 2007-006728 A
[Patent Document 4] JP 2002-510787 A

### [SUMMARY OF THE INVENTION]

### [PROBLEMS TO BE SOLVED BY THE INVENTION]

As mentioned above, various methods of collecting a nucleic acid have been investigated. As mentioned above, in the method described in Patent Document 4, it is expected that it is difficult to collect pre-miRNA and miRNA which are very short-chain nucleic acids. In addition, with respect to the collecting method using iron oxide as a support described in Patent Document 3, since no actual results of collecting a nucleic acid are described, it is not clear that nucleic acids of what sizes can be collected at what degree of efficiency. As the replication experiment of Patent Document 3, in Comparative Example 1 described below, the study for collecting a nucleic acid using iron oxide as a support was carried out, but it was shown that iron oxide is not suitable for collecting a nucleic acid because iron oxide exhibits very low adsorption for a nucleic acid. In Comparative Example 2, the experiment on collecting a nucleic acid using, as a support, cerium oxide without a polymer adsorbed on its surface on the assumption of an iron oxide support coated with a metal oxide was carried out. However, the nucleic acid adsorbed on cerium oxide could not be eluted efficiently.

The present inventors thought that it is necessary to find a support which adsorb a nucleic acid with high adsorption ratio and a method in which adsorbed nucleic acid can be eluted efficiently in order to collect a nucleic acid ranging from a very short nucleic acid such as pre-miRNA and miRNA to a long nucleic acid such as a genome.

### [MEANS FOR SOLVING THE PROBLEMS]

The present inventors have newly found that cerium oxide can function as a support having a high adsorption ratio of nucleic acids. The present inventors have discovered that the nucleic acid elution ratio can be improved without the decrease in the nucleic acid adsorption ratio by adsorbing a water-soluble neutral polymer on the surface of the support, thus the present invention has been completed.

### [EFFECT OF THE INVENTION]

The present invention allows for high-yield collection of a nucleic acid by an easy method when a support comprising a water-soluble neutral polymer adsorbed on a cerium oxide surface thereof is used, and further the high-yield collection of even a very short nucleic acid such as pre-miRNA or miRNA, which has been conventionally difficult to collect efficiently.

### [MODE FOR CARRYING OUT THE INVENTION]

The scope of the invention is defined by the appended claims.

For the biological sample used in the invention, any sample containing a nucleic acid(s) can be used. Examples of the nucleic acids include RNA, DNA, RNA/DNA (chimera) and artificial nucleic acids. Examples of DNAs include cDNA, micro DNA, cell-free DNA, genomic DNA, and synthetic DNA. Examples of RNAs include total RNA, mRNA, rRNA, miRNA, siRNA, snoRNA, snRNA or non-coding RNA, precursors thereof or synthetic RNA. Synthetic DNA and synthetic RNA can be prepared artificially based on a predetermined base sequence (it may be either native sequence or non-natural sequence) using, for example, an automated nucleic acid synthesizer.

Examples of biological samples include, but not limited to, cell-derived samples such as cultured cells, culture liquids of cultured cells, tissue samples and specimens; samples derived from microorganisms such as bacteria, fungi, protista and viruses; samples derived from animals including human such as body fluids such as blood, urine, saliva, mucous membranes, sweat, sputum and semen, and feces; and solutions containing a compound, which has a biological function, such as a protein, sugar, lipid in addition to a nucleic acid. The biological sample is preferably a cultured cell or a body fluid, and further preferably blood. Examples of blood include whole blood, plasma, serum, and blood cells.

When the biological sample is a liquid sample such as a body fluid, the present invention may be applied directly after the sample is collected, or a solution may be added after the sample is collected to dilute the liquid sample. When the biological sample is a solid sample such as a cell pellet or tissue fragment, the solid sample may be diluted with water or a buffer solution after being collected and then used in the present invention.

The biological sample may be subjected to a treatment as explained below if necessary. The treatment is carried out because the nucleic acid in a biological sample is often capsuled in a compound such as a cell membrane, a cell wall, a vesicle, a liposome, a micelle, a ribosome, a histone, a nuclear membrane, a mitochondrion, a virus capsid, an envelope, an endosome, and an exosome and because they often interact with each other. In order to collect a nucleic acid with a better yield, a treatment for releasing a nucleic acid from such compounds may be carried out.

Specifically, the following treatment may be performed to improve the collection efficiency of a nucleic acid from a biological sample containing *E. coli.* For example, a mixture solution of 0.2 M sodium hydroxide and 1% SDS may be added to the biological sample containing *E. coli* (alkaline denaturation method), or a 10% sarcosyl solution may be added to the biological sample containing *E. coli* (non-denaturation method by sarcosyl). Lysozyme may be added to these solutions. The sample may also be treated with proteinase K at 37°C for one hour. Other methods also include an sonication.

In order to improve the collection efficiency of a nucleic acid from a yeast-containing biological sample, the following treatment may be performed on the biological sample. For example, after the biological sample can be treated with Zymolyase commercially available from SEIKAGAKU CORPORATION or Nacalai Tesque Inc., followed by adding 10% SDS.

In order to improve the collection efficiency of a nucleic acid from a cell-containing biological sample, the biological sample can be subjected to the following treatment. For example, 1% SDS can be added. As another method, 4 M or more of guanidinium chloride, guanidine thiocyanate salt, urea or the like can be added. Sarcosyl may be added to this solution to a concentration of 0.5% or more. Mercaptoethanol may also be added to result in a concentration of 50 mM or more.

In the above operations, an inhibitor of a degradative enzyme for a nucleic acid may be added to suppress the degradation of the nucleic acid contained in the biological sample. As an inhibitor of DNase, EDTA may be added in a concentration of 1 mM or less. As an inhibitor of RNase, commercially available RNasin Plus Ribonuclease Inhibitor (Promega Corporation), Ribonuclease Inhibitor (TAKARA BIO INC.), RNase inhibitor (TOYOBO CO., LTD.), and the like can be used.

When DNA and RNA are present together in a biological sample, they can be separated by phenol-chloroform extraction. For example, when phenol-chloroform extraction is performed under acidic conditions, RNA and DNA are separated into an aqueous layer and a chloroform layer, respectively. Under the neutral conditions, RNA and DNA are distributed into an aqueous phase. This nature can be utilized to select the conditions depending on the type of the desired nucleic acid. The above-mentioned chloroform may be replaced by p-bromoanisole.

In the phenol-chloroform extraction, a commercially available reagent, ISOGEN (registered trademark: NIPPON GENE CO., LTD.), TRIzol (registered trademark: Life Technologies Japan Ltd.), RNAiso (TAKARA BIO INC.), or 3D-Gene (registered trademark) RNA extraction reagent from liquid sample kit (Toray Industries, Inc.) may be used. A single step in the treatments described above may be performed alone, or in combination with another step of a different operation. The concentration of the solution to be used may vary as appropriate.

In the present invention, as a solution containing a nucleic acid, a solution in which a nucleic acid, an artificial nucleic acid, or a nucleic acid modified with a dye, a phosphoric group or the like is dissolved, when a biological sample is used, a liquid sample such as a body fluid or a diluted solution thereof, or a diluted solution of a solid sample such as a cell pellet or tissue fragment can be used. A solution obtained after the biological sample containing a liquid sample or a solid sample is subjected to any of the above-mentioned treatments may be directly used or may be diluted as required. The solution to be used for dilution is not particularly limited, but a solution which is widely used with a solution containing a nucleic acid, such as water or a Tris-hydrochloride buffer solution is preferably used. A solution containing a nucleic acid is preferably a biological sample in which 4 M or more of guanidinium chloride, a guanidine thiocyanate salt, or urea is added.

In the present disclosure but not claimed, the length of a nucleic acid to be collected is not particularly limited, but nucleic acids within a range from short nucleic acids of several dozen bases in length to long nucleic acids such as a genome can be collected with high yield. Especially, short-chain nucleic acids of 1000 bases or less in length, which were conventionally difficult to be collected, can be also collected with a high yield, and pre-miRNAs and miRNAs of 100 bases or less in length can also be collected with a high yield.

In the present invention, the high-yield collection of a nucleic acid by using a support comprising a water-soluble neutral polymer adsorbed on a cerium oxide surface thereof can be achieved. The support according to the present invention is a support comprising a water-soluble neutral polymer adsorbed on a cerium oxide surface thereof. Thereinafter, it is described as the support according to the present invention.

In collecting a nucleic acid using the support according to the present invention, a nucleic acid is first adsorbed on the support according to the present invention and the support according to the present invention has an excellent capacity of adsorbing nucleic acids. The nucleic acid adsorption capacity of the support according to the present invention can be evaluated by the adsorption ratio of a nucleic acid adsorbed on the support according to the present invention, and calculated as follows. The amount of a nucleic acid in a solution containing the nucleic acid is first calculated. Subsequently, the support according to the present invention and the solution containing the nucleic acid are mixed, the amount of the nucleic acid in the mixture solution after the nucleic acid is adsorbed on the support according to the present invention is then calculated, and the difference from the amount of the nucleic acid in the solution containing the nucleic acid is obtained. The obtained value is used as the amount of the nucleic acid adsorbed on the support according to the present invention, and the adsorption ratio of the nucleic acid can be calculated by dividing the amount of the nucleic acid adsorbed on the support according to the present invention by the amount of the nucleic acid in the solution containing the nucleic acid. Examples of methods for quantifying the amount of a nucleic acid include a UV-vis absorbance measurement, a fluorescence measurement, a luminescence measurement, electrophoresis, PCR, RT-PCR, an analysis using a microarray, and an analysis using a sequencer. In case of an unmodified nucleic acid, the amount of the nucleic acid can be quantified by measuring the absorbance at 260 nm. In the case of a nucleic acid modified with a fluorescent dye, the amount of the nucleic acid can be quantified by comparing the fluorescence intensity derived from the fluorescent dye with the fluorescence intensity of a solution of a known concentration. Moreover, the quantification can be achieved by electrophoresis. In the method of calculating the collection ratio by electrophoresis, the collection ratio can be determined by migrating a sample collected simultaneously with a sample of a known concentration and then comparing a dye concentration of a band by image-analyzing after staining a gel.

On the other hand, in collecting a nucleic acid using the support according to the present invention, since the object of the present invention is to collecting a nucleic acid by eluting the nucleic acid adsorbed on the support according to the present invention from the support using an eluent, the support according to the present invention has excellent nucleic acid-eluting ability into an eluent. The elution capacity for a nucleic acid from the support according to the present invention can be evaluated as the elution ratio of the nucleic acid from the support according to the present invention into the eluent, and obtained as follows. An eluent is added to the support according to the present invention on which a nucleic acid is adsorbed, and the amount of the nucleic acid in the solution after the elution is calculated to obtain the elution amount of the nucleic acid. The elution ratio can be calculated by dividing this elution amount of the nucleic acid by the above-calculated amount of the nucleic acid adsorbed on the support according to the present invention.

The nucleic acid collection capacity in the method of collecting a nucleic acid according to the present invention can be evaluated as a nucleic acid collection ratio which is calculated as a product of the nucleic acid adsorption ratio and elution ratio calculated by the above method.

The nucleic acid collection capacity in the method of collecting a nucleic acid according to the present invention can be evaluated by comparing a collection quantity of a nucleic acid collected by the method according to the present invention with a collection quantity of the nucleic acid collected by a method other than the method according to the present invention without quantifying a collection quantity of a nucleic acid. Examples of the methods of collecting a nucleic acid other than the method according to the present invention include a collecting method by a commercially available kit. As a specific comparison method between collection quantities of a nucleic acid, a nucleic acid from the same amount of the respective biological samples is first adsorbed and eluted to be collected using the support according to the present invention and a support other than the support according to the present invention. The volumes of the eluents may be the same or different, preferably the same. When they are different, the collected nucleic acid is diluted or concentrated to the same volume. Both samples are electrophoresed, the gel is stained and the images are then acquired by a fluorescence scanner or the like, and then the band densities corresponding to nucleic acid fractions of interest are compared with each other by image analysis, whereby a collection quantity of a nucleic acid collected by the method according to the present invention can be compared with a collection quantity of the nucleic acid collected by a method other than the method according to the present invention.

In the present invention, a polymer is a general name for compounds in which a large number of repeating units, called a monomer which is a basic unit, are connected. The polymers used for the support according to the present invention include both of a homopolymer consisting of one kind of monomer and a copolymer composed of two or more kinds of monomers, as well as polymers with an arbitrary degree of polymerization. In addition, the polymers used for the support according to the present invention include both of naturally-produced polymers and synthetic polymers.

The water-soluble neutral polymer used for the support according to the present invention is a polymer which has water-soluble property and the solubility in water is at least 0.0001 wt% or more, preferably 0.001 wt% or more, more preferably 0.01 wt% or more, and further preferably 0.1 wt% or more.

The water-soluble neutral polymer used in the present invention is a polymer with a zeta potential of not less than -10 mV and not more than +10 mV in a solution of pH 7. More preferably, the water-soluble neutral polymer used in the present invention is a polymer with a zeta potential of not less than -8 mV and not more than +8 mV, further preferably not less than -7 mV and not more than +7 mV, and particularly preferably not less than -4.0 mV and not more than +6.5 mV.

The zeta potential is one of values indicating electrical properties on colloidal interfaces in a solution. When charged colloids are dispersed in a solution, on the surface of a colloid, an electrical double layer is formed by counter ions with respect to the charge of the colloidal surface. The electrical potential on this colloidal surface is called surface potential. Because the electrical double layer is formed by electrostatic interaction between the colloidal surface charges, ions are more strongly fixed as they are closer toward the colloid. In the electrical double layer, a layer in which counter ions are strongly fixed on the colloidal surface by electrostatic interaction is called a stern layer, and the potential of the stem layer is called a fixed potential. When a colloid is moved relative to a solution, the stem layer is also moved together with the colloid. In this case, there is a boundary surface that is moved together with the colloid outside the stem layer as viewed from the colloid due to the viscosity of the solution. This surface is called a slipping plane. The potential of this slipping plane is defined as a zeta potential when the potential at a point sufficiently far from the colloid is defined as zero. Thus, as the zeta potential varies depending on the colloidal surface charge and the surface charge changes according to protonation and deprotonation which depend on pH, the value in a solution of pH 7 is used as a standard in the present invention. Because the distance from the colloidal surface to the slipping plane is generally small compared to the colloidal size, the colloidal surface can be represented approximately as the slipping plane. In case of the water-soluble neutral polymer used in the present invention as well, the colloidal surface potential dispersed in the solution can be considered as the zeta potential.

For the zeta potential in the present invention, a value which is measured by laser Doppler electrophoresis is used. The measurement of zeta potentials can be performed using a zeta potential measurement instrument. The zeta potential measurement instruments are commercially available, for example, from Otsuka Electronics Co., Ltd., Malvern Instruments Ltd., Ranku Brother Ltd., PenKem Inc.

When the zeta potential of a polymer is measured, a polymer solution can be prepared as a colloidal dispersion to measure its zeta potential. For example, a polymer is dissolved in an electrolyte such as a phosphate buffer solution, a sodium chloride solution, and a citrate buffer solution to prepare a polymer solution, which is then measured by detecting scattered light and reflected light of the polymer dispersed in the solution. A bigger colloid size allows for the detection of scattered light and reflected light under a lower concentration.

Specific conditions for measuring the zeta potential of a polymer by a laser Doppler method is followings: a polymer is dissolved in a phosphate buffer solution (10 mM, pH 7) such that the concentration is not less than 0.1 wt% and not more than 10 wt%; this solution is then placed in a measuring cell; and it is installed in a zeta potential measurement instrument based upon laser Doppler electrophoresis as a principle to measure the zeta potential at room temperature. For measuring the zeta potential in the present invention, for example, ELS-Z manufactured by Otsuka Electronics Co., Ltd., can be used.

Specific water-soluble neutral polymers used for the support according to the present invention are selected from polyvinyl alcohol, polyethylene glycol, polyvinylpyrrolidone, poly(2-ethyl-2-oxazoline), polypropylene glycol, polyacrylamide, poly(N-isopropylacrylamide) or hydroxypropylmethylcellulose. Examples of further disclosed water-soluble polymers that may be used for the support include a polyvinyl polymer beyond polyvinyl alcohol or polyvinylpyrrolidone, a polyacrylamide polymer beyondpolyacrylamide or poly(N-isopropylacrylamide) such as poly(N-(hydroxymethyl)acrylamide), a polyalkylene glycol polymer beyond polyethylene glycol or polypropylene glycol such as polytetramethylene ether glycol, or a cellulose beyond poly(2-ethyl-2-oxazoline) or (hydroxypropyl)methylcellulose such as methylcellulose, ethylcellulose, 2-hydroxyethylcellulose, or hydroxypropylcellulose. Copolymers containing an above-mentioned polymer can be also used.

In addition, hydroxypropylmethylcellulose may also be included within the water-soluble neutral polymer in the present invention. Further polysaccharides or polysaccharide analogues such as ficoll, agarose, chitin and dextran as well as proteins and peptides such as albumin are also included within the water-soluble neutral polymer in the present disclosure but are not claimed.

A portion of functional groups of the water-soluble neutral polymer may be ionized or substituted with a functional group exhibiting positive or negative charge, or a functional group exhibiting solubility in water such as an acetyl group may be introduced to side chains.

A polymer of, as the molecular weight of the water-soluble neutral polymer, for example, preferably not less than 0.4 kDa and not more than 1000 kDa, more preferably not less than 2 kDa and not more than 500 kDa, more preferably not less than 4 kDa and not more than 150 kDa, even more preferably not less than 6 kDa and not more than 150 kDa, the most preferably not less than 6 kDa and not more than 10kDa, can be used.

A value which is measured by gel permeation chromatography (GPC) is herein used as a molecular weight. It can be measured using as an equipment a GPC equipment, for example, EcoSEC HLC-8320 GPC manufactured by Tosoh Corporation and using TSK-gelα column or the like.

The cerium oxide used as the support according to the present invention is an amphoteric oxide of the composition formula CeO₂, also referred to as ceria.

For the cerium oxide, naturally-produced cerium oxide or cerium oxide manufactured industrially may be used. Examples of the methods for producing cerium oxide include a method in which oxalate or carbonate salt of cerium is pyrolytically decomposed and a method in which the cerium hydroxide precipitate obtained by neutralizing an aqueous solution of nitrate salt of cerium is calcined. Cerium oxide manufactured industrially can be available from reagent manufacturers, catalyst chemical manufacturers, Japan Reference Catalyst of Catalysis Society of Japan, and the like.

For cerium oxide used for the support according to the present invention, granulated one is suitable. The particle size may be uniform, or those with different particle sizes may be combined in use. A particle size of cerium oxide is required to be such a size that cerium oxide particles precipitate in centrifuging a mixture of water and cerium oxide particles at 6000G for 1 minute and cerium oxide with the particle size of, for example, 100 µm or less, preferably 50 µm or less, more preferably 10 µm or less, can be used.

Disclosed but not claimed is that, the particle size can be calculated as a value of a 50% diameter (D50, a median diameter) obtained from a frequency distribution of the sphere equivalent diameter resulted by the measurement using a particle size analyzer based on laser diffraction scattering method.

The eluent used in the present invention is not particularly limited as long as a nucleic acid adsorbed on the support according to the present invention can be eluted, but is preferably a buffer solution, and the buffer solution may contain a chelating agent. Specific examples thereof include a citrate buffer solution containing citric acid and sodium citrate, a phosphate buffer solution containing phosphoric acid and sodium phosphate, and a Tris-EDTA buffer solution obtained by adding EDTA to a Tris-hydrochloric acid buffer solution containing tris(hydroxymethyl)aminomethane and hydrochloric acid.

The pH of the buffer solution is preferably pH 4 or more and pH 9 or less, more preferably pH 5 or more and pH 8 or less.

As a chelating agent contained in the buffer solution, a substance that has a ligand with plurality of coordination positions, and binds to a metal ion to form a complex can be used.

Specific examples of chelating agents include ethylenediaminetetraacetic acid (EDTA), nitrilotriacetic acid (NTA), glycol ether diamine tetraacetic acid (EGTA), polyphosphoric acid, metaphosphoric acid and/or salts thereof. The final concentration of the chelating agent is not particularly limited as long as it is 50 mM or more, and is preferably 100 mM or more, and further preferably 500 mM or more.

Examples of compounds as a chelating agent other than the above include anionic polymers. Since a polymer which has carboxylic acid on the side chains coordinate to a metal ion, the buffer solution may contain such a polymer. Examples of polymers having such a function include polyvinyl sulfonic acid and/or salt thereof. The final concentration is not particularly limited as long as it is 1 wt% or more, and preferably 10 wt% or more.

The present invention is a method of collecting a nucleic acid from a biological sample, comprising steps: a) mixing a support comprising a water-soluble neutral polymer adsorbed on a cerium oxide surface thereof with a solution containing a nucleic acid to adsorb the nucleic acid on the support; b) separating the support on which the nucleic acid was adsorbed from the solution mixed in the step a); and c) adding an eluent to the support on which the nucleic acid was adsorbed in step b) to collect the nucleic acid. Each step will be described in detail below.

The support used in the present invention is produced by adsorbing a water-soluble neutral polymer onto its cerium oxide surface. The polymer may not be necessarily adsorbed in an even thickness on the cerium oxide surface.

As a stage before the water-soluble neutral polymer is adsorbed, cerium oxide may be washed in advance with a solution such as water or ethanol to remove impurities adsorbed on the surface, or this washing operation may be omitted.

Examples of methods for adsorbing the water-soluble neutral polymer on the cerium oxide surface include a method in which the water-soluble neutral polymer is dissolved to prepare a water-soluble neutral polymer solution and bringing it into contact with cerium oxide. Specifically, cerium oxide may be dipped in the water-soluble neutral polymer solution, the water-soluble neutral polymer solution may be added dropwise to cerium oxide, the water-soluble neutral polymer solution may be coated on cerium oxide, and the water-soluble neutral polymer solution may be sprayed onto cerium oxide in the form of a mist.

The methods for dipping cerium oxide in the water-soluble neutral polymer solution are not particularly limited. For example, after the water-soluble neutral polymer solution was added to cerium oxide, the mixture may be allowed to stand or stirred. Examples of the methods of stirring include pipetting, mixing by inversion, and mixing by a disperser such as a stirrer, mixer, vortex or mill, or by an ultrasonicator.

The concentration of the water-soluble neutral polymer is not particularly limited, but preferably 0.01 wt% or more, more preferably, 0.1 wt% or more.

The dipping time in case of standing is not particularly limited, but preferably 5 minutes or more. The mixing time for stirring is not particularly limited as long as the water-soluble neutral polymer and the cerium oxide are mixed homogeneously, but in case of a vortex, it is preferably stirred for 1 minute or more, and preferably 5 minutes or more.

The water-soluble neutral polymer can also be dip-coated on the cerium oxide surface using a sifter or a sieve. The mixing time for dipping in the solution may be, in case of the polymer concentration of 0.1 wt% or more, 5 minutes or more, and preferably 30 minutes or more.

When the water-soluble neutral polymer solution is added dropwise, a dropper, a dropping funnel or the like can be used. When the polymer solution is added dropwise, the cerium oxide may also be shaken or rotated, or a spin coater or the like may be used.

When the water-soluble neutral polymer solution is coated, a brush, roller or a wire bar can be used.

When the water-soluble neutral polymer solution is sprayed in the form of a mist, an air spray, an air brush or the like can be used.

After the water-soluble neutral polymer is adsorbed on the cerium oxide by the methods as described above, a centrifugation operation may be carried out to remove the supernatant polymer solution, or the cerium oxide is directly used for collecting the nucleic acid without a centrifugation operation. When the water-soluble neutral polymer solution is dissolved in a solvent, after the water-soluble neutral polymer is adsorbed on the cerium oxide and the solvent is removed, it may be dried or may be used directly for collecting the nucleic acid without drying.

The obtained support according to the present invention may be stored and then used, or may be prepared at time of use.

When the obtained water-soluble neutral polymer is solid, the water-soluble neutral polymer solution can be prepared by dissolving the polymer in water or an organic solvent, and when the obtained water-soluble neutral polymer is a solution, the water-soluble neutral polymer solution can be prepared by diluting the solution. When it is hard to dissolve the polymer or mix the polymer due to the high viscosity of the solution, a heating treatment or sonication may be performed. As the organic solvent a solvent, such as ethanol, acetonitrile, methanol, propanol, tert-butanol, DMF, DMSO, acetone, ethylene glycol and glycerol, which is compatible with water is preferably used. When the polymer is poorly soluble in water, an organic solvent described above may be added.

A support produced by covalently binding the water-soluble neutral polymer to cerium oxide for example, through a linker molecule is not included in the support according to the present invention. Specific examples of linker molecules include silane coupling agents.

The step a) is a step of mixing the support according to the present invention prepared according to the above preparation method with a solution containing a nucleic acid to adsorb the nucleic acid on the support according to the present invention. The method of mixing the support according to the present invention with the solution containing a nucleic acid is not particularly limited, but may be carried out, for example, by pipetting or mixing by inversion, or using an instrument such as a mixer or vortex. The mixing time is not particularly limited, but may be about 5 minutes, or may be a longer time. The support according to the present invention may be packed in a column and a solution containing the nucleic acid may be allowed to pass through the column.

The step b) is a step of separating the support according to the present invention on which a nucleic acid was adsorbed from the solution mixed in the step a). Examples of separation methods include a method in which the solution mixed in the step a) is centrifuged, so that the support according to the present invention on which the nucleic acid is adsorbed is allowed to precipitate, and the supernatant is then removed. Since the relative gravity of the support according to the present invention on which the nucleic acid is adsorbed is higher than that of water, the support can be easily allowed to precipitate by the centrifugation operation. Conditions for centrifugation may be a treatment at 6000 G for 1 minute, and more preferably a treatment at 10000 G for 1 minute. Other separation methods include a method in which an ultrafiltration membrane is used. The solution mixed in the step a) is allowed to pass through an ultrafiltration membrane with a smaller pore size than the particle size of the support on which the nucleic acid is adsorbed, and the support on which the nucleic acid is adsorbed is then separated. Such an ultrafiltration membrane is available as a kit, and a centrifugal filter kit as typified by Ultrafree (registered trademark) manufactured by Merck Ltd., or Nanosep (registered trademark) manufactured by Pall Corporation can be obtained for use.

After the operation of the step b), the treatment as described below may be carried out if necessary. This is because that a biological sample-derived material other than the nucleic acid of interest may be adsorbed on the surface of the support according to the present invention after the step a). For example, washing or degradation treatment can be performed in order to isolate the nucleic acid in higher purity. Specifically, various treatments, can be performed, such as washing with water to remove non-specifically adsorbed compounds; washing with a surfactant to remove non-specifically adsorbed proteins; washing with a surfactant-containing solution to remove ions and low-molecular compounds; washing with an organic solvent to remove non-specifically adsorbed hydrophobic compounds; adding a protease to degrade non-specifically adsorbed proteins; adding a ribonuclease to isolate only DNA, and adding an DNA nuclease to isolate only RNA.

The step c) is a step of collecting the nucleic acid by adding an eluent to the support according to the present invention on which the nucleic acid was adsorbed which support was separated in the step b).

With respect to collecting the nucleic acid which was eluted by adding an eluent, when the support according to the present invention and the solution of the eluted nucleic acid are intended to be separated from each other, examples of such methods include a method of, in the step c), centrifuging a mixture obtained by adding the eluent to the support on which the nucleic acid was adsorbed to precipitate the support according to the present invention, and obtaining the supernatant in which the nucleic acid was eluted. Since the relative gravity of the support used in the present invention is greater than that of water, the support can be easily allowed to precipitate by the centrifugation operation. A centrifugation condition may be at 6000 G for 1 minute, preferably at 10000 G for 1 minute.

Other separation methods include a method in which an ultrafiltration membrane is used. The mixture obtained in the step c) is allowed to pass through an ultrafiltration membrane with a smaller pore size than the particle size of the support according to the present invention, which results in separating the support according to the present invention. Such an ultrafiltration membrane is available as a kit, and a centrifugal filter kit as typified by Ultrafree (registered trademark) manufactured by Merck Ltd., or Nanosep (registered trademark) manufactured by Pall Corporation can be obtained for use.

A nucleic acid thus collected can be chemically modified as necessary. Examples of chemical modifications include fluorescent dye modification, quencher modification, biotin modification, amination, carboxylation, maleinimidation, succinimidation, phosphorylation and dephosphorylation, to an end of a nucleic acid, and the other examples include intercalator staining. These modifications may be introduced by chemical reaction, or may be introduced by enzyme reaction. These modification group is introduced prior to the above-mentioned quantification, and the modification group introduced via chemical modification is quantified instead of quantifying collected nucleic acid itself, whereby the nucleic acid can be indirectly quantified. Since the present invention allows a nucleic acid to be collected, especially a short-chain nucleic acid to be collected with high yield, high sensitive quantification is possible in the above quantification.

The kit for collecting a nucleic acid according to the present invention can be used to efficiently collect a nucleic acid from a biological sample.

The kit for collecting a nucleic acid used according to the present invention includes the support used in the present invention and a buffer solution as components. The kit may further include an instruction and/or the like other than these.

The support used in the present invention included in the kit for collecting a nucleic acid according to the present invention may be in dry state, or in a state in which the support is dipped in a solution of a water-soluble neutral polymer.

As a buffer solution included in the kit for collecting a nucleic acid according to the present invention, a buffer solution which can be used as an eluent in the above step c) can be utilized.

### [EXAMPLES]

The present invention will be more specifically described by way of the following Examples.

### <Materials and Methods>

Polyethylene glycol was obtained from Merck Ltd.; poly(2-ethyl-2-oxazoline) was obtained from Alfa Aesar, A Johnson Matthey Company; and cerium oxide (particle size: 4.4 µm) was obtained from DAIICHI KIGENSO KAGAKU KOGYO CO., LTD. The aqueous polymer solutions used in Examples were obtained by dissolving polymers in water or a mixture solvent of water and ethanol at respective concentrations. Furthermore, unless otherwise specified, the cerium oxide was used for the experiments directly as purchased without a sieving process or the like.

Plasmid DNA (2.7 kbp): pUC 19 and *E. coli: E. coli* DH5α Competent Cells were purchased from TAKARA BIO INC.; ethidium bromide, LB medium: LB Miller, RPMI medium: RPMI 1640 (containing L-glutamine), and Zymolyase were purchased from Nacalai Tesque Inc.; Buffer RLT, Buffer RLT Plus, and DNeasy Blood & Tissue Kit were purchased from QIAGEN K.K.; MagMax cell-freeDNA Isolation kit and TrypLE Express were purchased from Thermo Fisher Scientific Inc.; fetal bovine serum: Fetal Bovine Serum Sterile Filtered was purchased from Equitech-Bio Inc.; and ferrosoferric oxide was purchased from KANTO CHEMICAL CO., INC. The YPD medium was prepared by mixing 1% Yeast extract (Becton, Dickinson and Company), 2% Polypeptone (Becton, Dickinson and Company) and 2% D-glucose (Wako Pure Chemical Industries, Ltd.). Synthesized products, as a very short-chain nucleic acid, obtained by converting a nucleic acid with 22 bases in length known as the let7a sequence of miRNA to a DNA sequence and to a RNA sequence were purchased from Eurofins Genomics K.K. Hereinafter, a synthetic nucleic acid of an RNA sequence will be referred to as RNA 22, and a synthetic nucleic acid of a DNA sequence as DNA 22. These nucleic acids were directly used without any particular purification. The DNA sequence of 566 bp used in the examples was obtained by PCR amplification. Hereinafter, it is referred to as DNA 566. *E. coli* genome fragment with 10 kbp or more in length was obtained from *E. coli* DH5α. Other reagents were purchased from Wako Pure Chemical Industries, Ltd., Tokyo Chemical Industry Co., Ltd., and Sigma-Aldrich Japan, and used directly as purchased without any particular purification.

CUTE MIXER CM-1000 manufactured by TOKYO RIKAKIKAI CO, LTD. was used as a mixer; Nanodrop 3300 manufactured by Thermo Fisher Scientific and FLUOROMAX-3 manufactured by HORIBA, Ltd. were used as a fluorometer; ELS-Z manufactured by Otsuka Electronics Co., Ltd. was used to measure a zeta potential; Mupid-eXU manufactured by ADVANCE CO., LTD. was used for agarose gel electrophoresis; and Mini Gel Slab Electrophoretic Device manufactured by AS ONE Corporation was used for acrylamide gel electrophoresis were used. The stained agarose gel was analyzed using a fluorescence scanner Typhoon 9410 manufactured by GE Healthcare Japan Corp. and the stained acrylamide gel was analyzed using a fluorescence scanner Typhoon FLA 9500 manufactured by GE Healthcare Japan Corp. The image analysis for the agarose gel was carried out using ImageQuant (registered trademark) manufactured by Molecular Dynamics and the image analysis for the acrylamide gel was carried out using ImageQuant TL (registered trademark) manufactured by Molecular Dynamics.

### <Comparative Example 1> Method of collecting a nucleic acid using only iron oxide as a support.

A method of collecting a nucleic acid using iron oxide as a support as described in Patent Document 3 was investigated. α-Iron oxide manufactured by Wako Pure Chemical Industries, Ltd. was used as iron oxide. In order to set the other to the same condition as in the example of Patent Document 3, 6 M guanidine thiocyanate aqueous solution and TE buffer solution or water were used as a solution for dissolving a nucleic acid and as an eluent, respectively.

First, iron oxide was weighed in an amount of 0.5 mg in a 1.5-ml tube. To each tube, 200 µl of ethanol was added, and each resulting mixture was vortex-mixed and then centrifuged for 1 minute by a centrifuge to remove the supernatant. This operation was further repeated twice to wash it, and the resulting material was used as a support.

Subsequently, 100 µl of a 6 M aqueous solution of a guanidine thiocyanate salt in which 100 pmol of DNA 22 was dissolved was added to the washed iron oxide, followed by stirring by a mixer for 5 minutes, and the resulting mixture was centrifuged (10000 G, 1 minute) to remove the supernatant. To the remaining support, 100 µl of 0.05% Tween water was added, and then vortexed. This operation was further repeated twice. Subsequently, 50 µl of water or TE (10 mM Tris-HCl, 1 mM EDTA, pH 8) buffer solution was individually added and then stirred by a mixer for 5 minutes. The resulting mixtures were centrifuged by a centrifuge (10000 G, 1 minute) and the supernatant was then collected as a nucleic acid solution.

The adsorption ratio of a nucleic acid on a support was calculated by the fluorescence measurement of the Cy3 as follows. First, a fluorescence intensity of 100 µl of the 6 M aqueous solution of a guanidine thiocyanate salt in which 100 pmol of DNA 22 was dissolved before adding cerium oxide was measured, and next the fluorescence intensity after adding and mixing cerium oxide was measured. The fluorescence intensity after adding cerium oxide was divided by the fluorescence intensity before adding cerium oxide, and the quotient was multiplied by the amount of the nucleic acid (100 pmol) before adding cerium oxide to calculate the amount of the nucleic acid in the solution. The difference between the amount of the nucleic acid (100 pmol) before adding cerium oxide and this value was taken to calculate the amount of the adsorbed nucleic acid. The amount of the adsorbed nucleic acid was divided by the amount of the nucleic acid (100 pmol) before adding cerium oxide to calculate an adsorption ratio.

An elution ratio was calculated by the fluorescence measurement of the Cy3 as follows. To cerium oxide on which the nucleic acid was adsorbed, 50 µl of a TE buffer solution was added, and a fluorescence of the eluent after the elution was measured. Subsequently, water and TE buffer solution in each of which 100 pmol of DNA 22 was dissolved were prepared, and fluorescences of these respective solutions were measured. The fluorescence intensity of the eluent was divided by the fluorescence intensity of the solution to calculate the amount of the eluted nucleic acid. The amount of the eluted nucleic acid was divided by the amount of the adsorbed nucleic acid to calculate the elution ratio. The collection ratio was calculated by multiplying the adsorption ratio to be calculated by the elution ratio. The results are shown in Table 1.

These results showed that, when iron oxide was used as a support, the nucleic acid adsorption ratio was very low and the nucleic acid elution was hardly observed. It was seen that iron oxide was not suitable for collecting a nucleic acid. The cited Patent Document 3 describes that a material coated with a polymer on its inorganic surface can be used for collecting a nucleic acid, but it is expected that coating an iron oxide surface with a polymer results in no change in adsorptive property to a nucleic acid. That is, it is expected that use of iron oxide coated with a polymer on its surface as a support results in a low nucleic acid collection ratio as with the result in Comparative Example 1.

**[Table 1]**

| Support | Bases in length | Adsorption solution | Eluent | Adsorptio n ratio [%] | Elution ratio [%] | Collection ratio [%] |
|---|---|---|---|---|---|---|
| Iron oxide | 22 | Guanidine thiocyanate | Water | 8 | 0 | 0 |
| | | | Tris-HCl | 11 | 0 | 0 |

### <Comparative Example 2> Method of collecting a nucleic acid using as a support cerium oxide with no water-soluble neutral polymer adsorbed on its surface.

Whether a nucleic acid can be collected efficiently using as a support cerium oxide with no water-soluble neutral polymer adsorbed on its surface was investigated. The same conditions and operations were carried out as in Comparative Example 1 except that cerium oxide was used as a support. The results are shown in Table 2.

These results showed that, when cerium oxide with no water-soluble neutral polymer adsorbed on its surface as a support, the nucleic acid adsorption ratio onto the cerium oxide support was very high, but it was seen that the nucleic acid elution ratio was low and therefore the nucleic acid could not be collected efficiently.

Patent Document 3 describes a method of further coating a magnetic inorganic material with a metal oxide, but it is expected that, when the metal oxide coated with cerium oxide on its surface is used as a support, the collection ratio is low as with the result in Comparative Example 2.

**[Table 2]**

| Support | Bases in length | Adsorption solution | Eluent | Adsorption ratio [%] | Elution ratio [%] | Collection ratio [%] |
|---|---|---|---|---|---|---|
| Cerium oxide | 22 | Guanidine thiocyanate | Water | 97 | 0 | 0 |
| | | | Tris-HCl | 98 | 0 | 0 |

### <Comparative Example 3> Preparation of a support comprising a water polymer other than a water-soluble neutral polymer adsorbed on a cerium oxide surface thereof.

Cerium oxide in an amount of 0.5 mg each was weighed into a 1.5-ml tube. As a polymer solution, polyacrylic acid (PAcA, 5.1 kDa, 10 wt%), polystyrene sulfonic acid (PSS, 7.5 kDa, 10 wt%), polyvinyl sulfonic acid (PVSA, 10 wt%), polyallylamine (PAA, 17 kDa, 10 wt%), and poly-L-lysine (PLL, 150 kDa, 1 wt%) in an amount of 50 µl each were individually added to the respective tubes, and respective mixtures were stirred for 10 minutes by a mixer. The mixtures were centrifuged by a centrifuge (10000 G, 1 minute), and the supernatant was removed to obtain the cerium oxide with respective polymers adsorbed.

### <Comparative Example 4> Method of collecting a nucleic acid using cerium oxide as a support on which a water-soluble polymer other than a water-soluble neutral polymer is adsorbed.

As a water-soluble polymer other than a water-soluble neutral polymer prepared in a 1.5-ml tube in Comparative Example 3, cerium oxide on which polyacrylic acid (PAcA, 5.1 kDa, 10 wt%), polystyrene sulfonic acid (PSS, 7.5 kDa, 1 wt%), polyvinyl sulfonic acid (PVSA, 10 wt%), polyallylamine (PAA, 17 kDa, 10 wt%), and poly-L-lysine (PLL, 150 kDa, 1 wt%) were individually adsorbed in an amount of 0.5 mg each were weighed and used as a support. A phosphate buffer solution (0.5 M, pH 8) was used as an eluent. The same operations were carried out as in Comparative Example 1 to calculate the nucleic acid collection ratio except the support and eluent. The results are shown in Table 3.

These results shows that, when the respective cerium oxide with polyacrylic acid, polyvinyl sulfonic acid and polystyrene sulfonic acid adsorbed are used as a support, the nucleic acid adsorption ratios and the elution ratio are both low and the nucleic acid collection ratios are also low. It resulted in that, when the respective cerium oxide with polyallylamine and poly-L-lysine adsorbed were used as a support, the adsorption ratio of nucleic acid was kept high, but the elution ratio was low and the collection ratio was also low.

### <Example 1> Preparation of a support comprising a water-soluble neutral polymer adsorbed on its cerium oxide surface.

Cerium oxide was weighed in an amount of 0.5 mg each in a 1.5-ml tube. As an aqueous polymer solution, water-soluble neutral polymers, i.e., polyvinyl alcohol (11% acetylation, PVA, 18 kDa, 10 wt%), poly(2-ethyl-2-oxazoline) (PEOz, 5 kDa, 10 wt%), polyethylene glycol (PEG, 10 kDa, 10 wt%), polyacrylamide (PAAm, 40kDa, 10 wt%), hydroxypropylmethylcellulose) (HPMC, 10 kDa, 10 wt%), and polyvinylpyrrolidone (PVP, 10 kDa, 10 wt%) in an amount of 50 µl each were individually added to these respective tubes. To the respective aqueous solutions of polypropylene glycol (PPG, 4 kDa, 10 wt%) and poly(N-isopropylacrylamide) (pNIPAAm, 30 kDa, 10 wt%), ethanol was added until the respective polymers were dissolved, and then each 50 µl of the respective mixtures was added to 0.5 mg of cerium oxide. The other conditions and operations were carried out in the same manner as in Comparative Example 3 to obtain cerium oxide supports on which respective polymers were adsorbed.

### <Example 2> Collection of a nucleic acid using a support comprising a water-soluble neutral polymer adsorbed on its cerium oxide surface.

The cerium oxide on which polyvinyl alcohol, poly(2-ethyl-2-oxazoline), polyethylene glycol, polypropylene glycol, polyacrylamide, poly(N-isopropylacrylamide), hydroxypropylmethylcellulose, and polyvinylpyrrolidone was individually adsorbed, as each water-soluble neutral polymer, which were produced in 1.5-ml tubes in Example 1, were weighed in an amount of 0.5 mg each and used as a support. Other conditions and operations were carried out in the same manner as in Comparative Example 4 to calculate the nucleic acid adsorption ratio, elution ratio, and collection ratio. The results are shown in Table 3.

These results showed that, in comparison with Comparative Example 4, when a support comprising a water-soluble neutral polymer adsorbed on its cerium oxide surface was used, the elution ratio and collection ratio are improved while the nucleic acid adsorption ratio remains high.

**[Table 3]**

| Polymer adsorbed on Cerium oxide | Adsorption ratio [%] | Elution ratio [%] | Collection ratio [%] | |
|---|---|---|---|---|
| PSS | 3 | 24 | 1 | Comparative Example 4 |
| PAcA | 14 | 10 | 1 | Comparative Example 4 |
| PVSA | 5 | 13 | 1 | Comparative Example 4 |
| PVA | 99 | 99 | 98 | Example 2 |
| PEOz | 99 | 85 | 84 | Example 2 |
| PEG | 98 | 92 | 92 | Example 2 |
| pNIPAAm | 95 | 60 | 57 | Example 2 |
| PAAm | 99 | 99 | 98 | Example 2 |
| HPMC | 98 | 96 | 94 | Example 2 |
| PVP | 99 | 95 | 94 | Example 2 |
| PPG | 98 | 95 | 93 | Example 2 |
| PAA | 95 | 0 | 0 | Comparative Example 4 |
| PLL | 93 | 0 | 0 | Comparative Example 4 |

### <Comparative Example 5> Measurement of a zeta potential of a water-soluble polymer other than a water-soluble neutral polymer.

Water-soluble polymers other than a water-soluble neutral polymer used in Comparative Example 4, *i.e.*, polyacrylic acid, polystyrene sulfonic acid, polyvinyl sulfonic acid, polyallylamine and poly-L-lysine were individually dissolved in a phosphate buffer solution (10 mM, pH 7) such that the final concentration would be not less than 0.1 wt% and not more than 10 wt%, and their zeta potentials were measured using ELS-Z manufactured by Otsuka Electronics Co., Ltd. The results are shown in Table 4. In Table 4, the zeta potentials obtained in this measurement and the collection ratios of DNA 22 when cerium oxide with the respective polymer adsorbed on the surface thereof was used as a support (results of Comparative Example 4) are correlated with each other, and the polymers were ordered in the ascending order of the zeta potential values.

These results showed that the zeta potentials of the water-soluble polymers other than the water-soluble neutral polymers used in Comparative Example 4 was not more than -17mV and not less than +11mY.

### <Example 3> Measurement of a zeta potential of a water-soluble neutral polymer

The water-soluble neutral polymers used in Example 2, i.e., polyvinyl alcohol, poly(2-ethyl-2-oxazoline), polyethylene glycol, polypropylene glycol, polyacrylamide, poly(N-isopropylacrylamide), hydroxypropylmethylcellulose, and polyvinylpyrrolidone were individually dissolved in a phosphate buffer solution (10 mM, pH 7) such that the final concentration would be not less than 1 wt% and not more than 10 wt%, and their zeta potentials were measured in the same manner as in Comparative Example 5. In Table 4, the zeta potentials obtained in this measurement and the collection ratios of DNA 22 when cerium oxide with the respective polymer adsorbed on the surface thereof was used as a support (results of Example 2) are correlated with each other, and the polymers were ordered in the ascending order of the zeta potential values.

These results showed that the zeta potentials of the water-soluble neutral polymers which had improved nucleic acid collection ratios in Example 2 were not less than -4mV and not more than +6.5mV in the solution of pH 7 and therefore improved collection ratio was achieved in comparison with the water-soluble polymers with the zeta potential of not more than -17mV and not less than +11mV.

**[Table 4]**

| Polymer adsorbed on Cerium oxide | Zeta potential [mV] | Collection ratio [%] | |
|---|---|---|---|
| PSS | -37 | 1 | Comparative Example 5 |
| PAcA | -41 | 1 | Comparative Example 5 |
| PVSA | -17 | 1 | Comparative Example 5 |
| PVA | -4.0 | 98 | Example 3 |
| PEOz | -2.7 | 84 | Example 3 |
| PEG | -1.2 | 92 | Example 3 |
| pNIPAAm | -1.4 | 57 | Example 3 |
| PAAm | -1.1 | 98 | Example 3 |
| HPMC | -0.89 | 94 | Example 3 |
| PVP | +1.1 | 94 | Example 3 |
| PPG | +6.5 | 93 | Example 3 |
| PAA | +11 | 0 | Comparative Example 5 |
| PLL | +14 | 0 | Comparative Example 5 |

### <Example 4> Relationship between elution and eluent of a nucleic acid adsorbed on a support comprising a water-soluble neutral polymer adsorbed on a cerium oxide surface thereof.

A support comprising polyethylene glycol adsorbed on a cerium oxide surface thereof was prepared according to Example 1 and weighed in an amount of 0.5 mg each in a 1.5-ml tube. As an eluent, 0.5 M citrate buffer solution (pH 5.6), 0.5 M phosphate buffer solution (pH 7.8), 0.5 M Tris-EDTA buffer solution (0.5 M, Tris-HCl, 0.5 M EDTA, pH 8) were individually used. Other conditions and operations were carried out in the same manner as in Comparative Example 1 to calculate the nucleic acid adsorption ratio, elution ratio and collection ratio. The results are shown in Table 5.

These results showed that the nucleic acid could be also collected with high yield by using any buffer solutions as an eluent.

**[Table 5]**

| Eluent | Adsorption ratio [%] | Elution ratio [%] | Collection ratio [%] |
|---|---|---|---|
| Citrate buffer solution (0.5 M, pH 5) | 99 | 58 | 58 |
| Citrate buffer solution (0.5 M, pH 6) | 99 | 70 | 70 |
| Phosphate buffer solution (0.5 M, pH 7) | 99 | 86 | 86 |
| Phosphate buffer solution (0.5 M, pH 8) | 98 | 92 | 92 |
| Tris-EDTA buffer solution (0.5 M, pH 8) | 99 | 74 | 72 |

### <Example 5> Relationship between a collection ratio and a length of a nucleic acid using a support comprising a water-soluble neutral polymer adsorbed on a cerium oxide surface thereof.

A support comprising polyethylene glycol adsorbed on a cerium oxide surface thereof was prepared according to Example 1, and weighed in an amount of 0.5 mg each in a 1.5-ml tube. As a solution containing a nucleic acid, 100 µl of a 6 M aqueous solution of guanidine thiocyanate in which 250 ng of DNA 566, 250 ng of pUC 19 (2.7 kbp), and 250 ng of *E. coli* genome fragment (> 10 kbp) were individually dissolved was used. Other conditions and operations were carried out in the same manner as in Comparative Example 3, to calculate the nucleic acid collection ratio. The results are shown in Table 6.

These results showed that the nucleic acids with any lengths could be collected efficiently by using a support comprising polyethylene glycol, which is a water-soluble neutral polymer, adsorbed on a cerium oxide surface thereof.

**[Table 6]**

| Bases in length | Collection ratio [%] |
|---|---|
| 566 bp | 99 |
| 2.7 kbp | 91 |
| 10 kbp or more | 84 |

### <Example 6> Collection of a nucleic acid from a fetal bovine serum.

A support comprising polyethylene glycol adsorbed on a cerium oxide surface thereof was prepared according to Example 1 and weighed in an amount of 2.5 mg each in a 1.5-ml tube. As solutions containing a nucleic acid, a mixture solution of 100 µl 6M guanidine thiocyanate salt aqueous solution in which 100 pmol of DNA 22 was dissolved and 100 µl of fetal bovine serum with a protein concentration of 30 mg/ml was used. Other conditions and operations were carried out in the same manner as in Comparative Example 4 to calculate the nucleic acid adsorption ratio, elution ratio, and collection ratio. The same experiment was performed for RNA 22 as well. The results are shown in Table 7. Note that the protein concentration in the collection liquid was the detection limit or less of the Bradford test (0.25 mg/ml or less).

These results showed that any of DNA 22 and RNA 22 could be also collected efficiently from a serum by using a support comprising polyethylene glycol adsorbed on a cerium oxide surface thereof.

**[Table 7]**

| Nucleic acid | Adsorption ratio | Elution ratio | Collection ratio [%] |
|---|---|---|---|
| DNA 22 | 99 | 93 | 92 |
| RNA 22 | 99 | 87 | 86 |

### <Example 7> Effect of the molecular weight of a water-soluble neutral polymer to be adsorbed on a cerium oxide surface.

Polyethylene glycol with a molecular weight of 6 kDa, 10 kDa, and 500 kDa and polyvinyl alcohol with a molecular weight of 18 kDa, 40 kDa, and 150 kDa (11% acetylated for any) were individually prepared to be 10 wt% and used as a polymer solution. A support comprising polyethylene glycol with each molecular weight adsorbed on a cerium oxide surface thereof was prepared in the same manner as in Example 1 and used as a support. Other conditions and operations were carried out in the same manner as in Comparative Example 4 to calculate the nucleic acid adsorption ratio, elution ratio, and collection ratio. The results are shown in Table 8.

These results showed that the nucleic acid could be collected using polymers with any molecular weight.

**[Table 8]**

| Adsorbed polymer | Molecular weight [kDa] | Adsorption ratio [%] | Elution ratio [%] | Collection ratio [%] |
|---|---|---|---|---|
| PEG | 6 | 99 | 94 | 93 |
| PEG | 10 | 98 | 92 | 92 |
| PEG | 500 | 92 | 53 | 49 |
| PVA | 18 | 99 | 81 | 80 |
| PVA | 40 | 99 | 70 | 69 |
| PVA | 150 | 97 | 77 | 75 |

### <Example 8> Relationship between the concentration and stirring time of the water-soluble neutral polymer in the method of producing the support according to the present invention

Cerium oxide was weighed in an amount of 0.5 mg each in a 1.5-ml tube. As a polymer aqueous solution, 50 µl of polyethylene glycol (PEG, 10 kDa), which is a water-soluble neutral polymer, was added to the respective tubes in concentrations of 0.1 wt%, 1 wt%, and 10 wt%, respectively. The mixtures with each concentration were stirred by a mixer for 1 minute, 5 minutes, and 30 minutes, respectively. The resulting mixtures were centrifuged by a centrifuge (10000 G, 1 minute), the supernatants were then removed, and the supports with polyethylene glycol adsorbed on their cerium oxide surface were thus obtained. Subsequent operations were carried out in the same manner as in Comparative Example 4 to calculate the nucleic acid collection ratio. The results are shown in Table 9.

These results showed that supports produced under any condition led to the result that the nucleic acid could be collected efficiently.

**[Table 9]**

| Mixing time | PEG concentration | Collection ratio [%] |
|---|---|---|
| 1 minute | 0.1 wt% | 51 |
| | 1 wt% | 53 |
| | 10 wt% | 93 |
| 5 minutes | 0.1 wt% | 70 |
| | 1 wt% | 83 |
| | 10 wt% | 91 |
| 30 minutes | 0.1 wt% | 75 |
| | 1 wt% | 89 |
| | 10 wt% | 99 |

### <Example 9> Relationship between the concentration and the dipping time of the water-soluble neutral polymer in the method of producing the support according to the present invention.

Cerium oxide was weighed in an amount of 0.5 mg each in a 1.5-ml tube. As a polymer aqueous solution, 50 µl of polyethylene glycol (PEG, 10 kDa), which is a water-soluble neutral polymer, was added to respective tubes in concentrations of 0.1 wt%, 1 wt%, and 10 wt%, respectively, and allowed to stand for 5 minutes and 30 minutes, respectively, without stirring operation such as pipetting. The resulting mixtures were centrifuged by a centrifuge (10000 G, 1 minute), the supernatants were then removed, and the supports with polyethylene glycol adsorbed on their cerium oxide surface were thus obtained. Subsequent operations were carried out in the same manner as in Comparative Example 4 to calculate the nucleic acid collection ratio. The results are shown in Table 10.

These results showed that supports produced under any condition led to the result that the nucleic acid could be collected efficiently.

**[Table 10]**

| Mixing time | PEG concentration | Collection ratio [%] |
|---|---|---|
| 5 minutes | 0.1 wt% | 62 |
| | 1 wt% | 74 |
| | 10 wt% | 80 |
| 30 minutes | 0.1 wt% | 71 |
| | 1 wt% | 69 |
| | 10 wt% | 92 |

### <Example 10> Relationship between the presence or absence and the collection ratio of a centrifugation operation in the method of producing the support according to the present invention.

Cerium oxide was weighed in an amount of 0.5 mg each in a 1.5-ml tube. As a polymer aqueous solution, 50 µl of polyethylene glycol (PEG, 10 kDa), which is a water-soluble neutral polymer, was added to the respective tubes in a concentration of 10 wt%. Subsequent operations in Example 10 were carried out without such operations as centrifugation by a centrifuge or removal of supernatants in Example 1. The operations were carried out in the same manner as in Comparative Example 4 except that the supports thus produced were used to calculate the nucleic acid adsorption ratio, elution ratio, and collection ratio, and the results are shown in Table 11.

These results showed that, in comparison with the results of the nucleic acid collection ratio in which polyethylene glycol was used as a water-soluble neutral polymer among the results in Example 2 in which the nucleic acid was collected using the supports produced in Example 1, both methods led to the result that the nucleic acid could be collected efficiently by producing the support according to the present invention.

**[Table 11]**

| Centrifugation | Adsorption ratio [%] | Elution ratio [%] | Collection ratio [%] | |
|---|---|---|---|---|
| Centrifuged | 98 | 92 | 92 | Example 2 |
| Not centrifuged | 99 | 94 | 93 | Example 10 |

### <Example 11> Collection of cell-free DNA from human plasma using the support according to the present invention.

In two 1.5-ml tubes, each 300 µl of human plasma was weighed, each 450 µl of Buffer RLT was added to the respective mixtures, and then the mixtures were mixed by pipetting. Among the resulting two samples, the genome was collected from a first sample using the support according to the present invention, and the genome was collected as control from a second sample using a commercially available kit.

First, 5 mg of cerium oxide with polyethylene glycol adsorbed produced in the same condition as in Example 1 was added to the first sample provided above, and mixed. The mixture was stirred by a mixer for 15 minutes and centrifuged (10000 G, 1minute), and then the supernatant was removed. To the remaining support, 400 µl of 0.05% Tween water was added, and then vortexed. This operation was further repeated twice. Subsequently, 50 µl of a phosphate buffer solution (0.5 M, pH 8) was added and the resulting mixture was stirred by a mixer for 30 minutes. The resulting mixtures were centrifuged by a centrifuge (10000 G, 1 minute) and the supernatant was then collected as a nucleic acid solution.

Subsequently, the cell-free DNA was collected from the second sample using a commercially available kit (MagMax cell-freeDNA Isolation kit, Thermo Fisher Scientific Inc.). For this, the volume of the eluent was 50 µl. The respective cell-free DNA eluents collected using the support according to the present invention and the commercially available kit were electrophoresed (10% acrylamide, 100 V, 35 minutes) and stained with SYBR Gold. The fluorescence images were measured using a fluorescence scanner and image analysis for the ratio of the band densities of cell-free DNA fractions (160 to 200 bp) was carried out. The ratio of collection quantities of the support according to the present invention and the commercially available kit was calculated instead of the collection ratio because the absolute quantity of the cell-free DNA contained in the human plasma was unknown. The results are shown in Table 12.

These results showed that use of the support according to the present invention led to the result that the cell-free DNA could be collected in 9.9-fold collection quantity of the commercially available kit even using human plasma as a biological sample.

**[Table 12]**

| Biological sample | Nucleic acid | Collection quantity (Ratio to a commercially available kit) | |
|---|---|---|---|
| Human plasma | Cell-free DNA | 9.9-fold | Example 11 |
| *E. coli* (DH5α) in the culture | Genome | 3.8-fold | Example 12 |
| Fission yeast (NBRC 1628) | Genome | 3.8-fold | Example 13 |
| Budding yeast (NBRC 0216) | Genome | 3.5-fold | Example 14 |
| Adherent cell (Panc 10.05) | Genome | 8.6-fold | Example 15 |
| Suspension cell (Jurkat) | Genome | 5.9-fold | Example 16 |

### <Example 12> Genome collection from E. coli (DH5α) using the support according to the present invention.

*E. coli* (DH5α) was cultured in a liquid medium of LB. For a genome collection, the *E. coli* concentration in the culture liquid was calculated with a value measured at OD₆₀₀. From the culture liquid, each 5 X 10⁷ cells was dispensed into two 1.5-ml tubes. Among the resulting two samples, the genome was collected from a first sample using the support according to the present invention, and the genome was collected from a second sample using a commercially available kit as control. The cell count of *E. coli* was calculated on the basis that it is 10⁹ cells/ml when OD₆₀₀ = 1.

First, 100 µl of Buffer RLT Plus was added to the first sample and vortexed. This solution was mixed with 5 mg of cerium oxide on which polyethylene glycol adsorbed produced in the same condition as in Example 1. The mixture was stirred by a mixer for 15 minutes and centrifuged (10000 G, 1 minute), and then the supernatant was removed. To the remaining support, 400 µl of 0.05% Tween water was added, and then vortexed. This operation was further repeated twice. Subsequently, 50 µl of a phosphate buffer solution (0.5 M, pH 8) was added and the resulting mixture was stirred by a mixer for 30 minutes. The resulting mixtures were centrifuged by a centrifuge (10000 G, 1 minute) and the supernatant was then collected as a nucleic acid solution.

Subsequently, the genome was collected from the second sample according to protocol using the commercially available kit (DNeasy Blood & Tissue Kit, QIAGEN K.K.). For this, the volume of the eluent was 50 µl. The genome eluents collected using the support according to the present invention and the commercially available kit was electrophoresed (1% agarose, 100 V, 60 minutes) and stained with ethidium bromide. The fluorescence images were measured using a fluorescence scanner and image analysis for the ratio of the band densities of genome fractions (near 25 kbp) was carried out. The ratio of collection quantities of the support according to the present invention and the commercially available kit was calculated instead of the collection ratio because the absolute quantity of the genome contained in the *E. coli* was unknown. The results are shown in Table 12.

These results showed that use of the support according to the present invention led to the result that the genome could be collected in 3.8-fold collection quantity of the commercially available kit even using *E. coli* (DH5α) in the culture liquid as a biological sample.

### <Example 13> Collection of a genome from a fission yeast (NBRC 1628) using the support according to the present invention.

A fission yeast (NBRC 1628) was cultured on a plate on which YPB medium was supplemented with 2% agarose, then transferred to a liquid medium of YPD and cultured. In relation to collecting the genome, the fission yeast concentration in the culture liquid was calculated by measuring the value at OD₆₀₀. From the culture liquid, each 5 X 10⁵ cells was dispensed into two 1.5-ml tubes. Among the resulting two samples, the genome was collected from a first sample using the support according to the present invention, and the genome was collected from a second sample using a commercially available kit as control. At this time, the cell count was calculated on the basis that it is 10⁷ cells/ml when OD₆₀₀ = 1.

First, 600 µl of Zymolyase solution (1 M sorbitol, 100 mM EDTA, 14 mM mercaptoethanol, 100 U/ml Zymolyase, pH 7.4) was added to the first sample and allowed to stand at 30°C for 30 minutes. Subsequently, the mixture was centrifuged (5000 G, 10 minutes), and then the supernatant was removed to obtain a pellet. To this, 200 µl of Buffer RLT Plus was added and vortexed. This solution was mixed with 5 mg of cerium oxide on which polyethylene glycol adsorbed produced in the same condition as in Example 1. The mixture was stirred by a mixer for 15 minutes and centrifuged (10000 G, 1 minute), and then the supernatant was removed. To the remaining support, 400 µl of 0.05% Tween water was added, and then vortexed. This operation was further repeated twice. Subsequently, 50 µl of a phosphate buffer solution (0.5 M, pH 8) was added and the resulting mixture was stirred by a mixer for 30 minutes. The resulting mixtures were centrifuged by a centrifuge (10000 G, 1 minute) and the supernatant was then collected as a nucleic acid solution.

Subsequently, the genome was collected from the second sample according to protocol using the commercially available kit (DNeasy Blood & Tissue Kit, QIAGEN K.K.). For this, the volume of the eluent was 50 µl. The genome eluents collected using the support according to the present invention and the commercially available kit was electrophoresed (1% agarose, 100 V, 60 minutes) and stained with ethidium bromide. The fluorescence images were measured using a fluorescence scanner and image analysis for the ratio of the band densities of genome fractions (near 25 kbp) was carried out. The ratio of collection quantities of the support according to the present invention and the commercially available kit was calculated instead of the collection ratio because the absolute quantity of the genome contained in the fission yeast was unknown. The results are shown in Table 12.

These results showed that use of the support according to the present invention led to the result that the genome could be collected in 3.8-fold collection quantity of the commercially available kit even using the fission yeast (NBRC 1628) as a biological sample.

### <Example 14> Collection of a genome from a budding yeast (NBRC 0216) using the support according to the present invention.

Other conditions and operations were carried out in the same manner as in Example 13 except that a budding yeast (NBRC 0216) was used in place of the fission yeast (NBRC 1628). The results are shown in Table 12.

These results showed that use of the support according to the present invention led to the result that the genome could be collected in 3.5-fold collection quantity of the commercially available kit even using the budding yeast (NBRC 0216) as a biological sample.

### <Example 15> Collection of a genome from an adherent cell (Panc 10.05) using the support according to the present invention.

An adherent cell (Panc 10.05) was cultured in a 25-cm² cell culture flask in which a RPMI medium was supplemented with fetal bovine serum so as to be 10% (v/v). For collecting a genome, the cell was peeled away from the culture flask using TrypLE Express. The peeled cell was transferred into a 15-ml tube and centrifuged (1500 rpm, 5 minutes, room temperature) to obtain a pellet, and the supernatant was removed by suction. Subsequently, 2 ml of a medium was placed and the mixture was resuspended. The number of cells was counted and each 5 x 10⁴ cells was dispensed from this cell suspension and transferred to two 1.5-ml tubes, centrifuged (300 G, 5 minutes) to obtain a pellet, and the supernatant was removed. Among the resulting two samples, the genome was collected from a first sample using the support according to the present invention, and the genome was collected from a second sample using a commercially available kit as control.

First, 100 µl of Buffer RLT Plus was added to the first sample and vortexed. This solution was mixed with 5 mg of cerium oxide on which polyethylene glycol adsorbed produced in the same condition as in Example 1. The mixture was stirred by a mixer for 15 minutes and centrifuged (10000 G, 1 minute), and then the supernatant was removed. To the remaining support, 400 µl of 0.05% Tween water was added, and then vortexed. This operation was further repeated twice. Subsequently, 50 µl of a phosphate buffer solution (0.5 M, pH 8) was added and the resulting mixture was stirred by a mixer for 30 minutes. The resulting mixtures were centrifuged by a centrifuge (10000 G, 1 minute) and the supernatant was then collected as a nucleic acid solution.

Subsequently, the genome was collected from the second sample according to protocol using the commercially available kit (DNeasy Blood & Tissue Kit, QIAGEN K.K.). For this, the volume of the eluent was 50 µl. The genome eluents collected using the support according to the present invention and the commercially available kit was electrophoresed (1% agarose, 100 V, 60 minutes) and stained with ethidium bromide. The fluorescence images were measured using a fluorescence scanner and image analysis for the ratio of the band densities of genome fractions (near 25 kbp) was carried out. The ratio of collection quantities of the support according to the present invention and the commercially available kit was calculated instead of the collection ratio because the absolute quantity of the genome contained in the adherent cell was unknown. The results are shown in Table 12.

These results showed that use of the support according to the present invention led to the result that the genome could be collected in 8.6-fold collection quantity of the commercially available kit even using the adherent cell (Panc 10.05) as a biological sample.

### <Example 16> Collection of a genome from a suspension cell (Jurkat) using the support according to the present invention.

A suspension cell (Jurkat) was cultured in a 25-cm² cell culture flask in which a RPMI medium was supplemented with fetal bovine serum so as to be 10% (v/v). For collecting a genome, the cell was transferred into a 15-ml tube and centrifuged (1500 rpm, 5 minutes) to obtain a pellet, and the supernatant was removed by suction. Two ml of a medium was placed and the mixture was resuspended. Other conditions and operations were carried out in the same manner as in Example 15. The results are shown in Table 12.

These results showed that use of the support according to the present invention led to the result that the genome could be collected in 5.9-fold collection quantity of the commercially available kit even using the suspension cell (Jurkat) as a biological sample.

### <Example 17> Collection of nucleic acids from urine.

A support comprising polyethylene glycol adsorbed on a cerium oxide surface thereof was prepared according to Example 1 and weighed in an amount of 1.5 mg in a 1.5-ml tube. As a solution containing a nucleic acid, 1 ml of artificial urine (0.2 g/l CaCl₂, 0.4 g/l MsSO₄, 8 g/l NaCl, 20 g/l urea, 0.3% ammonia) in which 100 pmol of DNA 22 was dissolved was prepared. To the artificial urine, 200 µl of 1 M acetate buffer solution (pH 4) was added and vortexed, and the mixture was added to the support. Other conditions and operations were carried out the same way as in Comparative Example 4, and the nucleic acid adsorption ratio, elution ratio, and collection ratio were calculated. The results are shown in Table 13.

These results showed that use of a support comprising polyethylene glycol adsorbed on cerium oxide surface thereof led to the result that DNA 22 could be collected efficiently even using urine as a biological sample.

**[Table 13]**

| Nucleic acid | Adsorption ratio | Elution ratio | Collection ratio [%] |
|---|---|---|---|
| DNA 22 | 99 | 70 | 69 |

### <Comparative Example 5> Method of collecting a nucleic acid using only ferrosoferric oxide as a support.

The same conditions and operations were carried out to collect a nucleic acid as in Comparative Example 1 except that ferrosoferric oxide was used in place of α-Iron oxide used in Comparative Example 1. The results are shown in Table 13.

These results showed that, when ferrosoferric oxide was used as a support, a nucleic acid was adsorbed, but a nucleic acid elution ratio was low. By combining the result of Comparative Example 5 with that of Comparative Example 1, it could be concluded that it was impossible to collect a nucleic acid efficiently when any kind of iron oxide was used as a support.

**[Table 14]**

| Support | Bases in length | Adsorption solution | Eluent | Adsorption ratio [%] | Elution ratio [%] | Collection ratio [%] |
|---|---|---|---|---|---|---|
| Ferrosoferric oxide | 22 | Guanidine thiocyanate | Water | 67 | 1.0 | 0.67 |
| | | | Tris-HCl | 64 | 2.8 | 1.8 |

### [Industrial Applicability]

The present invention allows for efficient collection of a nucleic acid ranging from a very short nucleic acid such as pre-miRNA or miRNA to a long nucleic acid such as a genome from a biological sample.

## Claims

1. A method of collecting a nucleic acid from a biological sample, comprising the steps of:
a) mixing a support comprising a water-soluble neutral polymer adsorbed on a cerium oxide surface thereof with a solution containing a nucleic acid to adsorb the nucleic acid to the support,
b) separating the support on which the nucleic acid was adsorbed from the solution mixed in the step a), and
c) collecting the nucleic acid by adding an eluent to the support on which the nucleic acid was adsorbed and which is separated in the step b),
wherein the water-soluble neutral polymer is polyvinyl alcohol, polyethylene glycol, polyvinylpyrrolidone, poly(2-ethyl-2-oxazoline), polypropylene glycol, polyacrylamide, poly(N-isopropylacrylamide) or hydroxypropylmethylcellulose.

2. The method of collecting a nucleic acid according to claim 1, wherein the watersoluble neutral polymer is a polymer with a zeta potential of not less than -10 mV and not more than +10 mV in a solution of pH 7.

3. The method of collecting a nucleic acid according to claim 1 or 2, wherein the eluent is a buffer solution.

4. The method of collecting a nucleic acid according to any one of claims 1 to 3, wherein the biological sample is blood, urine, saliva, a mucous membrane, sweat, a cultured cell, a culture liquid of cultured cells, a tissue sample, a specimen, a microorganism, a culture liquid of a microorganism or a virus.

5. Use of a support for collecting nucleic acid, the support comprising a water-soluble neutral polymer adsorbed on a cerium oxide surface thereof, wherein the water-soluble neutral polymer is polyvinyl alcohol, polyethylene glycol, polyvinylpyrrolidone, poly(2-ethyl-2-oxazoline), polypropylene glycol, polyacrylamide, poly(N-isopropylacrylamide) or hydroxypropylmethylcellulose.

6. The use according to claim 5, wherein the water-soluble neutral polymer is a polymer with a zeta potential of not less than -10 mV and not more than +10 mV in a solution of pH 7.

7. Use of a kit for collecting a nucleic acid, the kit comprising a support and a buffer solution, wherein the support comprises a water-soluble neutral polymer adsorbed on a cerium oxide surface thereof, wherein the water-soluble neutral polymer is polyvinyl alcohol, polyethylene glycol, polyvinylpyrrolidone, poly(2-ethyl-2-oxazoline), polypropylene glycol, polyacrylamide, poly(N-isopropylacrylamide) or hydroxypropylmethylcellulose.

8. The use according to claim 7, wherein the water-soluble neutral polymer is a polymer with a zeta potential of not less than -10 mV and not more than +10 mV in a solution of pH 7.

## Patentansprüche

1. Verfahren zum Sammeln einer Nukleinsäure aus einer biologischen Probe, das die folgenden Schritte umfasst:
a) Mischen eines Trägers, der ein an einer Ceriumoxidfläche adsorbiertes wasserlösliches neutrales Polymer umfasst, mit einer Lösung, die eine Nukleinsäure enthält, um die Nukleinsäure an den Träger zu adsorbieren,
b) Trennen des Trägers, an dem die Nukleinsäure adsorbiert wurde, von der in dem Schritt a) gemischten Lösung, und
c) Sammeln der Nukleinsäure durch das Hinzufügen eines Elutionsmittels zu dem Träger, an dem die Nukleinsäure adsorbiert wurde und der in dem Schritt b) getrennt wurde,
wobei das wasserlösliche neutrale Polymer Polyvinylalkohol, Polyethylenglycol, Polyvinylpyrrolidon, Poly(2-ethyl2-oxazolin), Polypropylenglycol, Polyacrylamid, Poly(N-isopro-pylacrylamid) oder Hydroxypropylmethylcellulose ist.

2. Verfahren zum Sammeln einer Nukleinsäure nach Anspruch 1, wobei das wasserlösliche neutrale Polymer ein Polymer mit einem Zetapotential von nicht weniger als -10 mV und nicht mehr als +10 mV in einer Lösung mit einem pH-Wert von 7 ist.

3. Verfahren zum Sammeln einer Nukleinsäure nach Anspruch 1 oder 2, wobei das Elutionsmittel eine Pufferlösung ist.

4. Verfahren zum Sammeln einer Nukleinsäure nach einem der Ansprüche 1 bis 3, wobei die biologische Probe Blut, Urin, Speichel, Schleimhaut, Schweiß, eine kultivierte Zelle, eine Kulturflüssigkeit von kultivierten Zellen, eine Gewebeprobe, eine medizinische Probe, ein Mikroorganismus, eine Kulturflüssigkeit eines Mikroorganismus oder ein Virus ist.

5. Verwendung eines Trägers zum Sammeln einer Nukleinsäure, wobei der Träger ein an einer Ceriumoxidfläche adsorbiertes wasserlösliches neutrales Polymer umfasst, wobei das wasserlösliche neutrale Polymer Polyvinylalkohol, Polyethylenglycol, Polyvinylpyrrolidon, Poly(2-ethyl2-oxazolin), Polypropylenglycol, Polyacrylamid, Poly(N-isopropylacrylamid) oder Hydroxypropylmethylcellulose ist.

6. Verwendung nach Anspruch 5, wobei das wasserlösliche neutrale Polymer ein Polymer mit einem Zetapotential von nicht weniger als -10 mV und nicht mehr als +10 mV in einer Lösung mit einem pH-Wert von 7 ist.

7. Verwendung eines Satzes zum Sammeln einer Nukleinsäure, wobei der Satz einen Träger und eine Pufferlösung umfasst, wobei der Träger ein an einer Ceriumoxidfläche adsorbiertes wasserlösliches neutrales Polymer umfasst, wobei das wasserlösliche neutrale Polymer Polyvinylalkohol, Polyethylenglycol, Polyvinylpyrrolidon, Poly(2-ethyl2-o-xazolin), Polypropylenglycol, Polyacrylamid, Poly(N-isopropylacrylamid) oder Hydroxypropylmethylcellulose ist.

8. Verwendung nach Anspruch 7, wobei das wasserlösliche neutrale Polymer ein Polymer mit einem Zetapotential von nicht weniger als -10 mV und nicht mehr als +10 mV in einer Lösung mit einem pH-Wert von 7 ist.

## Revendications

1. Procédé de collecte d'un acide nucléique à partir d'un échantillon biologique, comprenant les étapes consistant à :
a) mélanger un support comprenant un polymère neutre soluble dans l'eau adsorbé sur une surface en oxyde de cérium de celui-ci avec une solution contenant un acide nucléique afin d'adsorber l'acide nucléique sur le support,
b) séparer le support sur lequel l'acide nucléique était adsorbé de la solution mélangée dans l'étape a), et
c) collecter l'acide nucléique par ajout d'un éluant au support sur lequel l'acide nucléique était adsorbé et qui est séparé dans l'étape b),
dans lequel le polymère neutre soluble dans l'eau est l'alcool polyvinylique, le polyéthylène glycol, la polyvinylpyrrolidone, la poly(2-éthyl-2-oxazoline), le polypropylène glycol, le polyacrylamide, le poly(N-isopropylacrylamide) ou l'hydroxypropylméthylcellulose.

2. Procédé de collecte d'un acide nucléique selon la revendication 1, dans lequel le polymère neutre soluble dans l'eau est un polymère ayant un potentiel zêta non inférieur à -10 mV et non supérieur à +10 mV dans une solution à pH 7.

3. Procédé de collecte d'un acide nucléique selon la revendication 1 ou 2, dans lequel l'éluant est une solution tampon.

4. Procédé de collecte d'un acide nucléique selon l'une quelconque des revendications 1 à 3, dans lequel l'échantillon biologique est du sang, de l'urine, de la salive, une membrane muqueuse, de la sueur, une cellule cultivée, un liquide de culture de cellules cultivées, un échantillon de tissu, un spécimen, un micro-organisme, un liquide de culture d'un micro-organisme ou d'un virus.

5. Utilisation d'un support pour collecter un acide nucléique, le support comprenant un polymère neutre soluble dans l'eau adsorbé sur une surface d'oxyde de cérium de celui-ci, dans laquelle le polymère neutre soluble dans l'eau est l'alcool polyvinylique, le polyéthylène glycol, la polyvinylpyrrolidone, la poly(2-éthyl-2-oxazoline), le polypropylène glycol, le polyacrylamide, le poly(N-isopropylacrylamide) ou l'hydroxypropylméthylcellulose.

6. Utilisation selon la revendication 5, dans laquelle le polymère neutre soluble dans l'eau est un polymère ayant un potentiel zêta non inférieur à -10 mV et non supérieur à +10 mV dans une solution à pH 7.

7. Utilisation d'un kit pour collecter un acide nucléique, le kit comprenant un support et une solution tampon, dans laquelle le support comprend un polymère neutre soluble dans l'eau adsorbé sur une surface d'oxyde de cérium de celui-ci, dans laquelle le polymère neutre soluble dans l'eau est l'alcool polyvinylique, le polyéthylène glycol, la polyvinylpyrrolidone, la poly(2-éthyl-2-oxazoline), le polypropylène glycol, le polyacrylamide, le poly(N-isopropylacrylamide) ou l'hydroxypropylméthylcellulose.

8. Utilisation selon la revendication 7, dans laquelle le polymère neutre soluble dans l'eau est un polymère ayant un potentiel zêta non inférieur à -10 mV et non supérieur à +10 mV dans une solution à pH 7.
